# EUROPÄISCHE PATENTSCHRIFT

(11) **EP 0 470 925 B1**
(45) Veröffentlichungstag und Bekanntmachung des Hinweises auf die Patenterteilung: **04.08.1993**
(21) Anmeldenummer: 91810500.8
(22) Anmeldetag: 26.06.1991
(51) Int. Cl.: A61M 29/02

(54) **Dilatationskatheter**
Dilatation catheter
Cathéter de dilatation

(30) Priorität: 09.08.1990 CH 2596/90
(43) Veröffentlichungstag der Anmeldung: 12.02.1992
(73) Patentinhaber: SCHNEIDER (EUROPE) AG, 8180 Bülach (CH)
(72) Erfinder: Hofmann, Eugen, CH-8064 Zürich (CH); Pfenninger, Susi, CH-8610 Uster (CH); Niederhauser, Werner, CH-8046 Zürich (CH)
(74) Vertreter: Groner, Manfred

(56) Entgegenhaltungen:
- EP-A- 0 368 523
- US-A- 4 705 501

## Beschreibung

Die Erfindung betrifft einen Dilatationskatheter nach dem Oberbegriff des unabhängigen Patentanspruchs 1. Katheter dieser Gattung sind von A. Grüntzig zur perkutan transluminalen Rekanalisation chronischer Arterienverschlüsse erstmals entwickelt worden und haben seither stetig an Bedeutung gewonnen. Das hierzu verwendete Kathetersystem besteht in der Regel aus einem Führungsdraht, einem Dilatationskatheter mit einem wenigstens zweilumigen Schaft und einem distal an diesem angeordneten Dilatationsballon sowie einem Führungskatheter. Durch den letzteren wird der Führungsdraht und auf diesem der Dilatationskatheter so weit vorgeschoben, bis der Dilatationsballon sich in der Verengung befindet. Mit Flüssigkeit wird der Ballon nun dilatiert und das arteriosklerotische Material radial gegen und in die Gefässwandung gepresst, so dass nach dem Entfernen des Katheters der behandelte Bereich für die Gefässflüssigkeit wieder durchgängig ist.

Der Schaft des Dilatationskatheters besteht üblicherweise im wesentlichen aus zwei koaxialen Schlauchstücken, wobei der innere Schlauch den Führungsdraht aufnimmt und ein Zwischenraum zwischen den beiden Schlauchstücken das Lumen für die Flüssigkeit zum Dilatieren des Ballons bildet. Beim Vorschieben des Ballons in starke Verengungen wird infolge der vergleichsweise hohen axialen Kräfte der Ballon und der hinten mit diesem verbundene Schlauch gestaucht, was die Einführung des Ballons in die Verengung erschwert. Um dies zu vermeiden, wäre somit ein möglichst steifer Schaft erwünscht. Liegt die Verengung nach einer Gefässbiegung, so sollte hingegen der Schaft im vorderen Bereich möglichst flexibel sein, damit er mit möglichst geringer Reibung der Gefässbiegung folgen kann.

Um diese Schwierigkeit zu vermeiden, ist durch die EP-A-0 277 368, die zur Bildung des Oberbegriffs des Anspruchs 1 heraugezogen wurde, ein zweilumiger Dilatationskatheter bekannt geworden, bei welchem der Schaft aus mehreren Bereichen unterschiedlicher Steifigkeit hergestellt ist. Damit soll entlang des Katheters an unterschiedlichen Stellen optimale Flexibilität und Stabilität erreicht werden. Dazu sind Schaftabschnitte aus unterschiedlichen Werkstoffen hergestellt und diese miteinander thermoplastisch verbunden.

Weiter ist ein Dilatationsballon bekannt, bei dem die beiden koaxialen Schlauchstücke des Schaftes unmittelbar hinter dem Ballon mit Stegen verbunden sind. Diese Stege übertragen die axiale Schubkraft vom äusseren Schlauch auf den inneren Schlauch und sollen das oben genannte Stauchen des Schaftes bei der Behandlung stark stenosierter Gefässe verhindern. Solche Stege erschweren und verteuern die Herstellung des Katheters wesentlich und verengen das Lumen für den Durchlass der Druckflüssigkeit.

Der Erfindung liegt die Aufgabe zugrunde, einen Dilatationskatheter der genannten Gattung zu schaffen, der die oben genannten Schwierigkeiten vermeidet und der insbesondere auch zur Behandlung von stark stenosierten Gefässabschnitten geeignet ist, die sich in oder nach einer Gefässbiegung befinden.

Die Aufgabe wird durch die Erfindung gemäss Anspruch 1 gelöst.

Der proximale Abschnitt des Schaftes besitzt, im Längsschnitt gesehen, vier Wandungen, während der distale Abschnitt für die beiden Lumina lediglich drei Wandungen erfordert. Bei gleichem oder ähnlichem Kunststoff ist der proximale Abschnitt steifer als der distale Abschnitt.

Durch die Verwendung unterschiedlich steifer Kunststoffe kann der Unterschied in der Steifigkeit der beiden Abschnitte beinahe beliebig gewählt werden. Der flexiblere distale Abschnitt ist vorzugsweise in seiner Länge an die Länge des gebogenen Gefässabschnitts angepasst. Ist der Dilatationsballon in die Engstelle eingesetzt, so verläuft der flexiblere distale Abschnitt im gebogenen Bereich des Gefässes und der steifere proximale Abschnitt im weitgehend geraden Bereich des Gefässes. Bei einer koronaren Stenose ist der distale Abschnitt beispielsweise 15 - 25 cm und der proximale Abschnitt etwa 110 cm lang. Der längere proximale Abschnitt gewährleistet durch den runden Querschnitt des inneren Schlauchstücks ein reibungsarmes Gleiten des Führungsdrahtes. Der distale Abschnitt verhindert eine Stauchung des Ballons, da in diesem Abschnitt die Wandungen der beiden Lumina nicht getrennt sind, und sich diese somit in Längsrichtung nicht gegeneinander verschieben können.

Nach einer Weiterbildung der Erfindung überlappen sich die beiden Abschnitte an ihren Verbindungsstellen und sind an den anliegenden Flächen miteinander verschweisst. In diesem Fall bestehen die Abschnitte aus einem thermoplastischen Kunststoff, beispielsweise aus einem Polyamid, Polyurethan, Polyvinylchlorid, Polyester, Polyaethylen, oder einem anderen geeigneten Kunststoff. Beide Abschnitte können aus dem gleichen Kunststoff oder aus unterschiedlichen, miteinander verschweissbaren Kunststoffen bestehen.

Weist hierbei wenigstens ein Abschnitt an dem mit dem anderen Abschnitt verbundenen Ende eine verminderte Wandstärke auf, so lässt sich weitgehend verhindern, dass der Schaft an seiner genannten Verbindungsstelle härter ist als in anderen Bereichen. Die verminderte Wandstärke wird vorteilhafterweise durch eine Streckung des bzw. der betreffenden Enden erreicht.

Ein Ausführungsbeispiel des erfindungsgemässen Dilatationskatheters wird nachfolgend anhand der Zeichnungen näher erläutert. Es zeigen:
- Fig. 1: eine Ansicht eines erfindungsgemässen Dilatationskatheters,
- Fig. 2: einen Längsschnitt durch einen Abschnitt des Schaftes,
- Fig. 2a: die Enden zweier Schaftabschnitte im Längsschnitt und vor dem Zusammenfügen dieser beiden Abschnitte,
- Fig. 3: ein Querschnitt entlang der Linie III-III in Fig. 1,
- Fig. 4: ein Querschnitt entlang der Linie IV-IV in Fig. 1,
- Fig. 5: ein Querschnitt entlang der Linie V-V in Fig. 1 und 2,
- Fig. 6: im Längsschnitt ein Blutgefässegment mit einem eingeführten Dilatationskatheter, und
- Fig. 7: ein Längsschnitt durch das vordere Ende des Dilatationskatheters und durch das vordere Ende eines Führungsdrahtes.

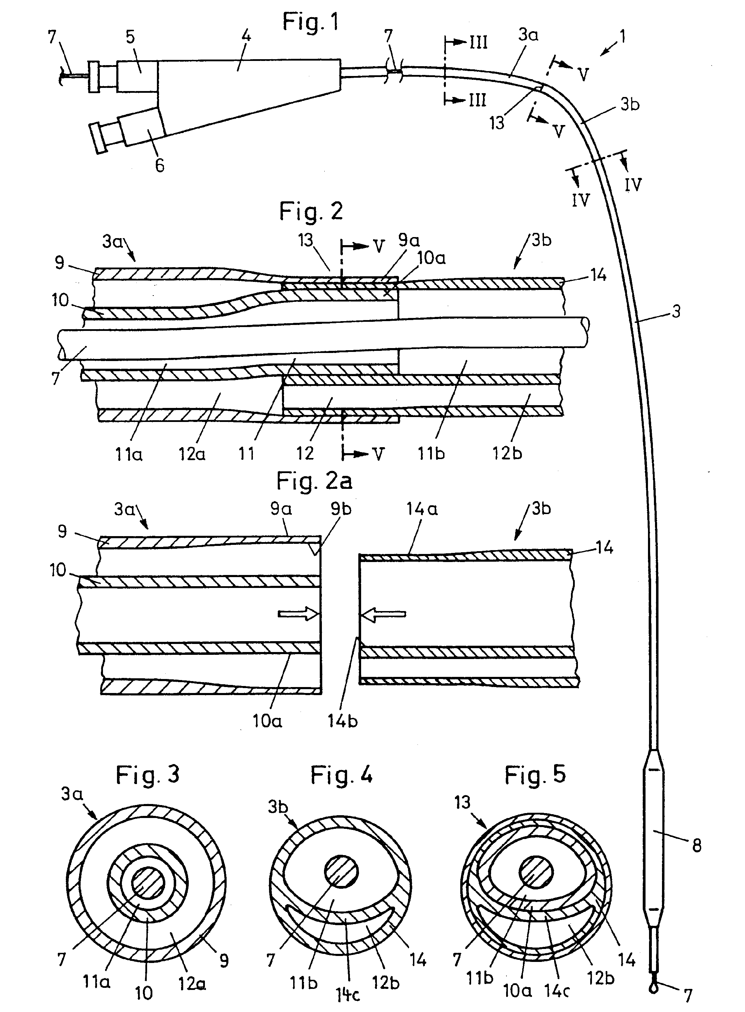

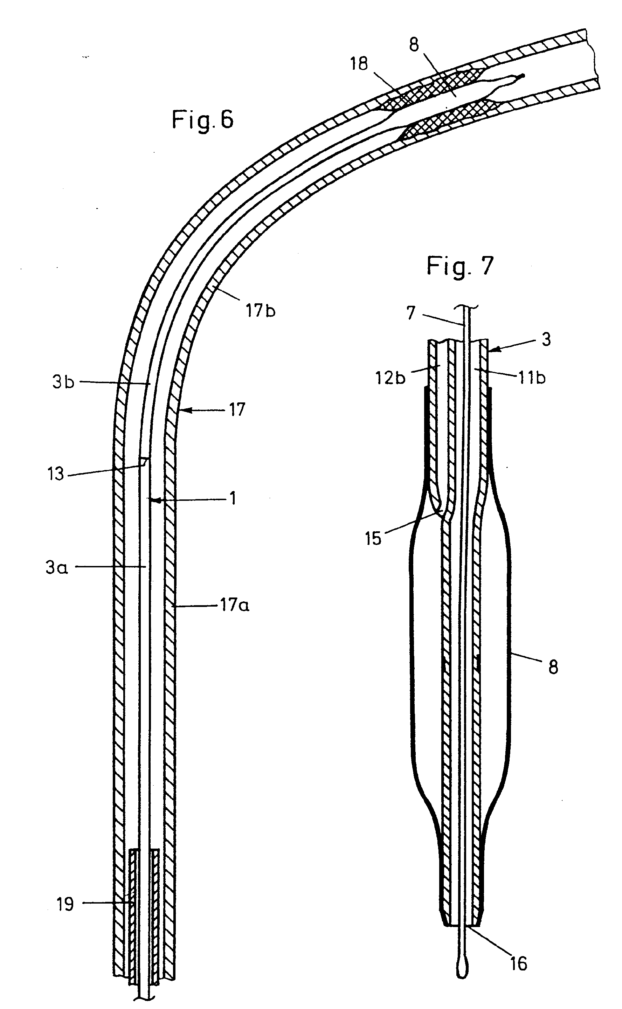

Der in Fig. 1 gezeigte Dilatationskatheter 1 wird durch einen Führungskatheter 19 (Fig. 6) in ein Blutgefäss 17 eingeführt, bis sich der Dilatationsballon 8 vor der zu behandelnden Engstelle 18 befindet. Mit Hilfe eines Führungsdrahtes 7 wird der Ballon 8,wie in Fig. 6 gezeigt, in den Durchgang der Engstelle 18 eingeschoben. Der Führungsdraht 7 ist in einem durchgehenden ersten Lumen 11 des Dilatationskatheters 1 in Längsrichtung verschiebbar. Der Innenraum des Ballons 8 ist über ein zweites Lumen 12 mit einer hier nicht gezeigten Druck-Saugeinrichtung verbunden, die an einem Anschluss 6 eines Verbindungsstückes 4 angeschlossen ist. Ein zweiter Anschluss 5 des Verbindungsstückes 4 dient zur Einführung und Abdichtung des Führungsdrahtes 7. Das erste Lumen 11 des Dilatationskatheters 1 ist somit mit dem Anschluss 5 und das zweite Lumen 12 mit dem Anschluss 6 verbunden.

Ein flexibler Schaft 3 ist an seinem distalen Ende mit dem Ballon 8 und an seinem proximalen Ende mit dem Verbindungsstück 4 verbunden. Die Figur 7 zeigt den allgemein bekannten Ballon 8, der über eine Oeffnung 15 mit dem zweiten Lumen 12 und über diesen mit dem Anschluss 6 verbunden ist. Mittels der genannten Druck-Saugeinrichtung kann der Ballon 8 zu seiner Einführung in das Gefäss 17 gefaltet und zur Behandlung der Stenose 18 dilatiert werden. Der Führungsdraht 7 kann wie in Fig.7 gezeigt, an seinem distalen Ende durch eine Oeffnung 16 über den Dilatationsballon 8 hinaus vorgeschoben werden. Er ist wie bekannt an seinem distalen Ende besonders flexibel und dennoch torsionssteif.

Der Schaft 3 ist aus einem proximalen Abschnitt 3a und einem distalen Abschnitt 3b hergestellt. Diese Abschnitte 3a und 3b sind separat hergestellt und an der Verbindungsstelle 13 miteinander verbunden. Wie die Figuren 3 und 4 zeigen, sind die Querschnitte der beiden Abschnitte 3a und 3b verschieden. Der proximale Abschnitt 3a besteht aus zwei koaxialen Schlauchstücken 9 und 10, wobei das innere Schlauchstück 10 ein Lumen 11a für den Führungsdraht 7 bildet. Der Zwischenraum zwischen den Schlauchstücken 9 und 10 bildet ein Lumen 12a, in welchem Druckflüssigkeit zwischen dem Ballon 8 und der Druck-Saugpumpe zirkulieren kann. Die Schlauchstücke 9 und 10 besitzen einen kreisrunden Querschnitt und bestehen aus einem thermoplastischen Kunststoff. Der Schaftabschnitt 3a ist in bekannter Weise mit dem Verbindungsstück 4 fest verbunden.

Der Schaftabschnitt 3b besteht aus einem zweilumigen, extrudierten Schlauchstück 14, das in bekannter Weise mit dem Ballon 8 verbunden ist. Ein erstes annähernd kreisförmiges Lumen 11b dient zur Aufnahme des Führungsdrahtes 7 und ein zweites halbmondförmiges Lumen 12b dient zur Aufnahme der Druckflüssigkeit. Beide Lumen sind durch eine am Abschnitt 3b angeformte Trennwand 14c voneinander getrennt.

Die Abschnitte 3a und 3b sind gemäss Fig. 2 derart miteinander verbunden, dass die Lumen 11a und 11b das erste Lumen 11 und die Lumen 12a und 12b das Lumen 12 bilden.

Die Lumen 11 und 12 sind somit auch an der Verbindungsstelle 13 vollständig voneinander getrennt. Um die separat hergestellten Abschnitte 3a und 3b miteinander zu verbinden, werden diese zusammengeschoben, bis sich die entsprechenden Enden auf einer Länge von wenigen Millimetern überlappen. Wie die Fig. 2a zeigt, ist vorzugsweise das Schlauchstück 9 an dem zu verbindenden Ende in Längsrichtung gestreckt und weist einen Bereich 9a mit verminderter Wandstärke auf. Dadurch ist vermieden, dass der Aussendurchmesser des Schaftes 3 an der Verbindungsstelle 13 wesentlich grösser ist als ausserhalb dieses Bereiches.

Im Bereich der Verbindungsstelle 13 sind die beiden Schlauchabschnitte 3a und 3b vorzugsweise miteinander verschweisst. Vorzugsweise ist die Aussenfläche 14a des Schlauchstücks 14 mit der Innenfläche 9b des Schlauchstückes 9 und die Aussenseite 10a des Schlauchstückes 10 mit der Innenseite 14b des Schlauchstückes 14 verschweisst. Möglich ist auch eine Ausführung, bei welcher das Schlauchstück 14 an dem zu verbindenden Ende eine verminderte Wandstärke aufweist. Diese verminderte Wandstärke kann hier beispielsweise auch durch Abschleifen des Schlauchstückes 14 an der Aussenseite 14a erfolgen. Denkbar ist auch eine Ausführung, bei welcher die beiden Abschnitte 3a und 3b am überlappenden Bereich 13 miteinander verklebt sind.

Sind die Abschnitte 3a und 3b aus gleichem oder ähnlichem Kunststoff hergestellt und sind die Wandstärken annähernd gleich, so besitzt das Schlauchstück 3a eine grössere Steifigkeit als das Schlauchstück 3b. Der Hauptgrund der unterschiedlichen Steifigkeit wird darin gesehen, dass das Schlauchstück 3a im Längsschnitt gemäss Fig. 2 aus vier Wandbereichen und der Abschnitt 3b lediglich aus drei Wandbereichen besteht. Die unterschiedliche Steifigkeit ist somit in erster Linie durch die unterschiedlichen Strukturen der beiden Abschnitte 3a und 3b verursacht.

Die Länge des flexibleren Abschnittes 3b ist vorzugsweise an die Länge des bogenförmigen Abschnittes des zu behandelnden Blutgefässes 17 angepasst, wie dies in Fig. 6 schematisch gezeigt ist. Die Gefässverengung 18 befindet sich hier in Strömungsrichtung der Gefässflüssigkeit gesehen in oder nach einer Gefässbiegung 17b und diese wiederum schliesst an einen weitgehend geraden Gefässabschnitt 17a an. Bei der Einführung des Dilatationskatheters 1 in das Gefäss und insbesondere bei der Einführung des Ballones 8 in die Engstelle 18 kann der flexiblere Abschnitt 3b der Biegung des Gefässes 17 mit geringer Verletzungsgefahr leicht folgen. Da der Abschnitt 3b den in Fig. 4 gezeigten Querschnitt aufweist, ist eine Stauchung des Ballones 8 in Längsrichtung auch dann nicht zu erwarten, wenn die Engstelle 18 einer Verschiebung des Katheters vergleichsweise hohen Widerstand bietet. In dem steifen aber weitgehend gerade verlaufenden Abschnitt 3a gleitet der Führungsdraht 7 besonders leicht, da das Lumen 11a weitgehend kreisrund ist. Dies kann durch geeignete Beschichtungen des Führungsdrahtes 7 und der Innenseite des Schlauchstückes 10 noch verbessert werden. Der in Fig. 6 gezeigte Gefässverlauf ist bei vielen Behandlungsfällen typisch. Denkbar ist auch eine Ausführung, bei welcher beide Abschnitte 3a und 3b oder einer dieser Abschnitte mehr als zweilumig sind. So kann beispielsweise der Abschnitt 3b vierlumig sein.

## Patentansprüche

1. Dilatationskatheter zur perkutanen transluminalen Rekanalisation chronischer Arterienverschlüsse, mit einem Schaft (3) und einem an diesem distal angeordneten Dilatationsballon (8), einem in Längsrichtung durchgehenden ersten Lumen (11) für einen Führungsdraht (7) und einem im Ballon (8) endenden zweiten Lumen (12) für eine Druckflüssigkeit, wobei der Schaft (3) aus wenigstens zwei separat hergestellten und miteinander verbundenen Abschnitten (3a, 3b) besteht, dadurch gekennzeichnet, daß ein proximaler Abschnitt (3a) aus zwei koaxialen Schlauchstücken (9, 10) und ein flexiblerer distaler Abschnitt (3b) aus einem zweilumigen Schlauchstück (14) hergestellt sind und die Abschnitte (3a, 3b) derart miteinander verbunden sind, dass das erste Lumen (11b) des distalen Abschnitts (3b) mit dem Lumen (11a) des koaxialen inneren Schlauchstücks (10) des proximalen Abschnitts (3a) und das zweite Lumen (12b) des distalen Abschnitts (3b) mit dem Lumen (12a) zwischen den beiden koaxialen Schlauchstücken (9, 10) des proximalen Abschnitts (3a) verbunden ist.

2. Dilatationskatheter nach Anspruch 1, dadurch gekennzeichnet, dass die beiden Abschnitte (3a, 3b) sich an ihrer Verbindungsstelle (13) überlappen und an den anliegenden Flächen miteinander verschweisst sind.

3. Dilatationskatheter nach Anspruch 1 oder 2, dadurch gekennzeichnet, dass wenigstens ein Abschnitt (3a) an dem mit dem anderen Abschnitt (3b) verbundenen Ende eine verminderte Wandstärke aufweist.

4. Dilatationskatheter nach Anspruch 3, dadurch gekennzeichnet, dass das entsprechende Ende des distalen Abschnitts (3a) in Längsrichtung gestreckt ist.

5. Dilatationskatheter nach Anspruch 2 oder 3, dadurch gekennzeichnet, dass an der Verbindungsstelle (13), das innere Schlauchstück (10) des proximalen Abschnitts (3a) in das erste Lumen (11b) des distalen Abschnitts eingesetzt ist, und dass das zweite Lumen (12a) des proximalen Abschnitts (3a) mit dem zweiten Lumen (12b) des distalen Abschnitts (3b) verbunden ist.

6. Dilatationsballon nach Anspruch 1, dadurch gekennzeichnet, dass die beiden Abschnitte (3a, 3b) miteinander verklebt sind.

7. Dilatationskatheter nach einem der Ansprüche 1 bis 6, dadurch gekennzeichnet, dass das zweite Lumen (12) im Bereich des proximalen Abschnittes (3a) einen etwa kreisförmigen querschnitt und im Bereich des distalen Abschnitts (3b) des Schafts (3) einen halbmondförmigen querschnitt aufweist.

## Claims

1. A dilatation catheter for the percutaneous transluminal recanalization of chronic arterial occlusions, having a shaft (3) and a dilating balloon (8) arranged distally on said shaft, a first lumen (11), passing through in the longitudinal direction, for a guide wire (7) and a second lumen (12), terminating in the balloon (8), for a pressure fluid, the shaft (3) comprising at least two sections (3a, 3b) produced separately and connected to each other, characterised in that a proximal section (3a) consists of two coaxial hose pieces (9, 10) and a more flexible distal section (3b) consists of a two-lumen hose piece (14), and that the sections (3a, 3b) are connected to each other such that the first lumen (11b) of the distal section (3b) is connected to the lumen (11a) of the coaxial inner hose piece (10) of the proximal section (3a) and the second lumen (12b) of the distal section (3b) is connected to the lumen (12a) between the two coaxial hose pieces (9, 10) of the proximal section (3a).

2. A dilatation catheter according to claim 1, characterised in that the two sections (3a, 3b) overlap at their point of connection (13) and are welded together on the adjoining faces.

3. A dilatation catheter according to claim 1 or claim 2, characterised in that at least one section (3a) has a reduced wall thickness at the end connected to the other section (3b).

4. A dilatation catheter according to claim 3, characterised in that the corresponding end of the distal section (3a) is stretched in the longitudinal direction.

5. A dilatation catheter according to either claim 2 or 3, characterised in that, at the point of connection (13), the inner hose piece (10) of the proximal section (3a) is inserted into the first lumen (11b) of the distal section, and that the second lumen (12a) of the proximal section (3a) is connected to the second lumen (12b) of the distal section (3b).

6. A dilating balloon according to claim 1, characterised in that the two sections (3a, 3b) are bonded to each other.

7. A dilatation catheter according to any one of claims 1 to 6, characterised in that the second lumen (12) has a substantially circular cross-section in the region of the proximal section (3a) and a crescent-shaped cross-section in the region of the distal section (3b) of the shaft (3).

## Revendications

1. Cathéter à dilatation pour la recanalisation transluminale percutanée d'occlusions artérielles chroniques, avec une tige (3) et un ballon de dilatation (8) disposé à une position distale sur cette tige, une première lumière (11) traversante en direction longitudinale pour un fil de guidage (7) et une seconde lumière (12) se terminant dans le ballon (8) pour un liquide sous pression, la tige (3) étant constituée d'au moins deux parties (3a, 3b) réalisées séparément et mutuellement assemblées, **caractérisé** en ce qu'une partie proximale (3a) est constituée de deux tronçons coaxiaux de tuyau souple (9, 10), et une partie distale plus flexible (3b) est constituée d'un tronçon de tuyau souple (14) à deux lumières, et les parties (3a, 3b) sont mutuellement assemblées de telle sorte que la première lumière (11b) de la partie distale (3b) est reliée à la lumière (11a) du tronçon de tuyau souple coaxial intérieur (10) de la partie proximale (3a), et la seconde lumière (12b) de la partie distale (3b) est reliée à la lumière (12a) présente entre les deux tronçons coaxiaux de tuyau souple (9, 10) de la partie proximale (3a).

2. Cathéter à dilatation selon la revendication 1, **caractérisé** en ce que les deux parties (3a, 3b) se chevauchent en leur lieu d'assemblage (13) et sont assemblées par soudage sur les faces en application mutuelle.

3. Cathéter à dilatation selon la revendication 1 ou 2, **caractérisé** en ce qu'au moins une partie (3a) présente une épaisseur de paroi réduite à son extrémité assemblée à l'autre partie (3b).

4. Cathéter à dilatation selon la revendication 3, **caractérisé** en ce que l'extrémité correspondante de la partie distale (3b) est étirée en direction longitudinale.

5. Cathéter à dilatation selon la revendication 2 ou 3, **caractérisé** en ce qu'au lieu d'assemblage (13), le tronçon de tuyau souple intérieur (10) de la partie proximale (3a) est introduit dans la première lumière (11b) de la partie distale, et en ce que la seconde lumière (12a) de la partie proximale (3a) est reliée à la seconde lumière (12b) de la partie distale (3b).

6. Cathéter à dilatation selon la revendication 1, **caractérisé** en ce que les deux parties (3a, 3b) sont mutuellement assemblées par collage.

7. Cathéter à dilatation selon l'une quelconque des revendications 1 à 6, **caractérisé** en ce que la seconde lumière (12) présente une section approximativement circulaire dans la région de la partie proximale (3a), et une section en forme de croissant dans la région de la partie distale (3b) de la tige (3).
